(19) Europäisches Patentamt
European Patent Office
Office européen des brevets

(11) **EP 2 138 232 A1**

(12) **EUROPEAN PATENT APPLICATION**

(43) Date of publication:
**30.12.2009 Bulletin 2009/53**

(51) Int Cl.:
***B01L 1/00*** (2006.01)    ***C12Q 1/68*** (2006.01)

(21) Application number: **08158781.8**

(22) Date of filing: **23.06.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventor: **The designation of the inventor has not yet been filed**<br><br>(74) Representative: **Damen, Daniel Martijn**<br>**Philips**<br>**Intellectual Property & Standards**<br>**P.O. Box 220**<br>**5600 AE Eindhoven (NL)** |
| (71) Applicant: **Koninklijke Philips Electronics N.V.**<br>**5621 BA Eindhoven (NL)** | |

(54) **Detection of tapped aliquots of amplified nucleic acids**

(57) The present invention relates to the amplification and detection of amplified nucleic acid sequences employing methods and devices with distinct temperature zones, particularly amplification zone and hybridizations zones. The methods and devices of the present invention may be used for quantitative analysis of target nucleic acid sequences, for simultaneous quantitative analysis of multiple target nucleic acid sequences or for analyzing a sample for the presence of a target nucleic acid.

Fig 2:

**Description**

FIELD OF THE INVENTION

The present invention relates to a process for conducting amplification of nucleic acids, particularly PCR, and to a device for conducting the method of the present invention. The invention is especially suited for the simultaneous identification and quantification of nucleic acids present in a sample, e.g. a biological sample. Further, the present invention relates to a device for amplification and quantitative real-time detection of target nucleic acid sequences in an amplification reaction, particularly in a polymerase chain reaction (PCR), in an amplification solution in a reaction container comprising at least two kind of zones that are thermally decoupled from each other, wherein one zone is a surface hybridization zone having a substantially constant temperature allowing for hybridization of oligonucleotide probes to nucleic acid sequences, wherein an inner surface of the surface hybridization zone is coated with capture probes for target nucleic acid sequences.

BACKGROUND OF THE INVENTION

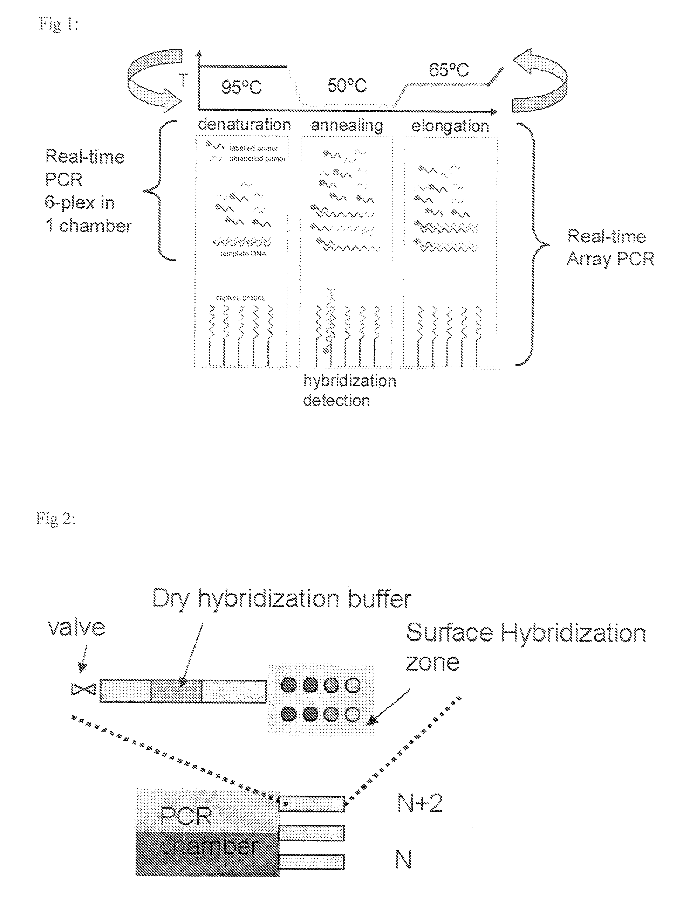

Amplification of nucleic acid sequences can be performed using a variety of amplification reactions, among them and most prominent the polymerase chain reaction (PCR). PCR is a method for amplification of specific nucleic acid sequences. PCR is an enzymatic reaction that primarily takes place in solution. When the amplification process is monitored in real-time, PCR can be used for quantitative analysis. Real-time PCR normally uses fluorescent reporters, such as intercalating dyes, TaqMan probes, Scorpion primers, molecular beacons. When more than one nucleic acid target sequence is to be analyzed, two approaches can be taken. The first approach is to parallelize the reactions, i.e. to run each reaction in a separate compartment. The second approach is to multiplex the reactions, i.e. to run the reactions in the same compartment and to use different fluorophore reporters for each reaction. This approach is limited by the number of fluorophores that can efficiently be discriminated. The current state-of-the-art is that six reactions can be multiplexed. Appended Fig. 1 illustrates current single-chamber multiplex-PCR and array PCR concepts.

The thermal requirements of the PCR process and of the surface hybridization process are very different. The PCR amplification process requires (fast) temperature cycling wherein part of the cycle the sample is above the dsDNA melting temperature. On the other hand the surface hybridization needs to take place at a temperature below the nucleic acid melting point, and for optimum performances diffusion limited local depletion of amplicons above the capture probe cites should be avoided.

The present multiple-zone concept allows decoupled optimization of these two processes, i.e. the amplification and the surface hybridization.

SUMMARY OF THE INVENTION

The present invention relates to a method and device for the amplification and detection of target nucleic acid sequences using distinct temperature zones. In particular, the present invention relates to a method for amplification and detection of target nucleic acid sequences in an amplification solution (which possibly contains nucleic acids with the target nucleic acid sequences) in a reaction container, comprising the steps of (a) providing a reaction container comprising at least one surface hybridization zone in which capture probes are immobilized on a surface, wherein said capture probes are substantially complementary to regions on said target nucleic acid sequences; (b) adding the amplification solution to said reaction container; (c) generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences; (d) performing an amplification of target nucleic acid sequences in the reaction container; and (e) detecting amplified nucleic acid sequences in periodic or defined intervals during and/or after amplification, wherein amplified nucleic acid sequences are detected by hybridization of capture probes to said amplified nucleic acid sequences in said surface hybridization zone.

In a preferred embodiment the present invention relates to a method for amplification and detection of target nucleic acid sequences in an amplification solution in a reaction container, comprising the steps of:

providing a reaction container comprising at least one surface hybridization zone in which capture probes are immobilized on a surface, wherein said capture probes are substantially complementary to regions on said target nucleic acid sequences; adding the amplification solution to said reaction container; generating a temperature zone profile in the reaction container with at least two kinds of thermally decoupled zones, wherein one kind of zone is substantially identical to the surface hybridization zones, wherein the surface hybridization zones have a temperature allowing for hybridization of the capture probes to the target nucleic acid sequences, and the other kind of zone has (a) temperature(s) allowing for amplification of the target nucleic acid sequences; performing an amplification of target nucleic acid sequences in the amplification zone; transferring an aliquot of said amplification solution at periodic or defined intervals during and/or after amplification from the amplification zone to said surface hybridization zone; and
detecting amplified nucleic acid sequences in periodic or defined intervals during and/or after amplification, wherein amplified nucleic acid sequences are detected by hybridization of capture probes to said amplified nucleic acid sequences in said surface hybridization zone.

The present invention also relates to a device for amplification and detection of target nucleic acid sequences in an amplification solution in a reaction container, comprising
a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences and at least one other kind of zone;
one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences;
a detection system that detects target nucleic acid sequences which are bound to said capture probes but does essentially not detect target nucleic acid sequences which are not bound to said capture probes; and
transportation system for transporting the amplification solution between the zones.

The device of the present invention may preferably be a device for amplification and detection of target nucleic acid sequences in an amplification solution in a reaction container, comprising:

a reaction container comprising a first compartment for amplification of target nucleic acid sequences and one or more second compartment(s) comprising a surface hybridization zone, wherein capture probes that are substantially complementary to regions on said target nucleic acid sequences are immobilized on a surface in said surface hybridization zone and wherein;
a surface-specific detection system that detects targets which are bound to said surface but does essentially not detect targets which are in solution;
one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences; and
a transportation system for transporting the amplification solution between the zones.

It is preferred that the reaction container is comprised in an exchangeable cartridge.
Also within the scope of the present invention is the use of the methods, cartridges and devices of the present invention for quantitative analysis of target nucleic acid sequences, for simultaneous quantitative analysis of multiple target nucleic acid sequences or for analyzing a sample for the presence of a target nucleic acid. Preferably, the methods and devices described herein are used for clinical diagnosis, point-of care diagnosis, bio-molecular diagnostics, gene or protein expression arrays, environmental sensors, food quality sensors or forensic applications. The present invention also relates to the use of the methods, cartridges and devices of the present invention in real-time PCR or real-time multiplex PCR.

BRIEF DESCRIPTION OF THE DRAWINGS

Fig. 1: Illustration of steps during real-time array PCR, which comprises 3 temperature steps per cycle, similar to real-time PCR. The difference between real-time array PCR illustrated in the illustrative example of Fig. 1 and real-time PCR in general is that in real-time array PCR the annealing step is combined with a hybridization step. The hybridization step may also be performed at a lower temperature than the annealing temperature, resulting in a four-step temperature profile.
Fig. 2: Schematic view of the main embodiment. Lower part: thermocycler zone (left) and surface hybridization zone (right). Upper part: Enlarged view of Surface hybridization zone with valve and dried hybridization buffer in outlet.
Fig. 3: Top view and cross sectional view (along A-A) of a cartridge according to the invention, with:

(50) Cartridge
(51) Body housing of the cartridge, which comprises an at least partly transparent material to enable optical detection. The cartridge is preferably made of (but is not limited to) plastic and like materials or glass. The cartridge can also be an assembly of a transparent substrate with capture probes and a box containing fluid channels.
(20) Sites (typically spots) with capture probes that are substantially complementary to target nucleic acid sequences. Typically, but by no means limiting, the capture probe sites are spots 0.1-0.3 mm in diameter. (20-I) a capture probe site that is also visible in the cross-sectional view along line A-A.
(40) Inlet for fluid.
(41) Thermocycler zone.
(42) Valve.
(43) Surface hybridization zone.

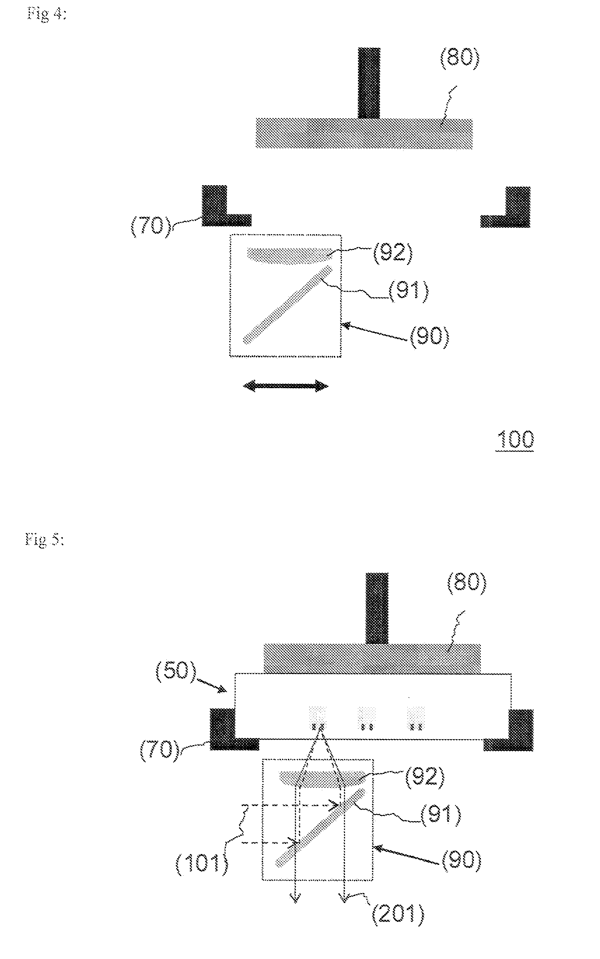

Fig. 4: Device (100) according to the present invention without the cartridge comprising the reaction chamber. The device is ready to receive a (disposable) cartridge with the reaction chamber. The device comprises:

(70) Sample holder for cartridge (50)
(80) Heaters for defining the temperature in the zones (1)-(3) of the cartridge, for this particular case there will be three heaters (80) to define the temperatures in zones (1), (2), (3).
(90) Detection system ("reader") for detecting the target nucleic acids that are hybridized to the capture probes (20). A simplified schematic illustration is shown of a scanning unit for confocal detection. Where the scanning unit comprises an illumination/collection (of the fluorescence) lens/objective (92) and a dichroic mirror (91) that reflects the excitation light (101 in Fig. 5) resulting in excitation light directed towards the cartridge. The same lens (92) collects the fluorescence (201 in Fig. 5) generated by the labeled target molecule and directs this fluorescence via the dichroic mirror (91), which transmits the fluorescent light, towards a detector (not shown here).

Fig. 5: Device of Fig. 4 with cartridge (50) comprising the reaction chamber. The numbering is identical to the numbering of Fig. 4.

DETAILED DESCRIPTION OF EMBODIMENTS

The present invention relates to a method and device for the amplification and detection of target nucleic acid sequences using distinct temperature zones. In particular, the present invention relates to a method for amplification and detection of target nucleic acid sequences in an amplification solution (which possibly contains nucleic acids with the target nucleic acid sequences) in a reaction container, comprising the steps of (a) providing a reaction container comprising at least one surface hybridization zone in which capture probes are immobilized on a surface, wherein said capture probes are substantially complementary to regions on said target nucleic acid sequences; (b) adding the amplification solution to said reaction container; (c) generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences; (d) performing an amplification of target nucleic acid sequences in the reaction container; and (e) detecting amplified nucleic acid sequences in periodic or defined intervals during and/or after amplification, wherein amplified nucleic acid sequences are detected by hybridization of capture probes to said amplified nucleic acid sequences in said surface hybridization zone.

In a preferred embodiment the present invention relates to a method for amplification and detection of target nucleic acid sequences in an amplification solution in a reaction container, comprising the steps of:

providing a reaction container comprising at least one surface hybridization zone in which capture probes are immobilized on a surface, wherein said capture probes are substantially complementary to regions on said target nucleic acid sequences;
adding the amplification solution to said reaction container;
generating a temperature zone profile in the reaction container with at least two kinds of thermally decoupled zones, wherein one kind of zone is substantially identical to the surface hybridization zones, wherein the surface hybridization zones have a temperature allowing for hybridization of the capture probes to the target nucleic acid sequences, and the other kind of zone has (a) temperature(s) allowing for amplification of the target nucleic acid sequences;
performing an amplification of target nucleic acid sequences in the amplification zone;
transferring an aliquot of said amplification solution at periodic or defined intervals during and/or after amplification from the amplification zone to said surface hybridization zone; and
detecting amplified nucleic acid sequences in periodic or defined intervals during and/or after amplification, wherein amplified nucleic acid sequences are detected by hybridization of capture probes to said amplified nucleic acid sequences in said surface hybridization zone.

The present invention also relates in a particular aspect to a method for amplification and detection of target nucleic acid sequences in an amplification solution in a reaction container, comprising the steps of (a) providing a reaction container comprising at least two separated compartments, wherein one compartment comprises an amplification zone and at least one other compartment comprises the at least one surface hybridization zone, (b) inserting an amplification solution into the amplification zone, (c) performing an amplification of target nucleic acid sequences in the amplification zone, (d) transferring an aliquot of said amplification solution at periodic or defined intervals during and/or after amplification from the amplification zone to said surface hybridization zone, and (e) detecting the amplified nucleic acid sequences in the aliquoted amplification solution in the surface hybridization zone, wherein amplified nucleic acid sequences are detected by hybridization of capture probes to said amplified nucleic acid sequences and wherein said hybridization takes place in said surface hybridization zone and said capture probes are immobilized on a surface and are substantially complementary to regions on said target nucleic acid sequences.

Thus, the invention relates in a preferred embodiment to a method for amplification and detection of target nucleic acid sequences in an amplification solution in a reaction container, comprising the steps of (i) providing a reaction container comprising one compartment for amplification of the target nucleic acid sequences and at least one compartment for surface hybridization and detection; (ii) inserting an amplification solution into the amplification compartment; (iii) performing an amplification of target nucleic acid sequences in the amplification compartment; (iv) transferring an aliquot of said amplification solution at periodic or defined intervals during and/or after amplification from the amplification compartment to the surface hybridization and detection compartment; and (v) detecting the amplified nucleic acid sequences in the aliquoted amplification solution in the surface hybridization and detection compartment, wherein amplified nucleic acid sequences are detected by hybridization of capture probes to said amplified nucleic acid sequences and wherein said hybridization takes place in said surface hybridization and detection compartment and said capture probes are immobilized on a surface and are substantially complementary to regions on said target nucleic acid sequences.

A further particular embodiment of the invention relates to a method for amplification and detection of target nucleic acid sequences in an amplification solution in a reaction container comprising the steps of the above-mentioned methods, wherein amplification of target nucleic acid sequences in a polymerase chain reaction is performed in one or more thermally decoupled thermocycler zones and wherein the temperature in said thermocycler zone is cycled between a temperature allowing for denaturation of the target nucleic acid, a temperature allowing for primer annealing to said target nucleic acid and a temperature allowing for primer extension, wherein said target nucleic acid serves as a template, and wherein an aliquot of the amplification solution is transferred from said thermocycler zone to a surface hybridization zone in periodic or defined intervals during or after amplification.

In preferred embodiments of the methods of the present invention, the amplification zone is connected to one or more surface hybridization zones through one or more outlets comprising a valve, wherein opening of the valve or the tap results in a transfer of an aliquot from the thermocycler zone to a surface hybridization zone.

A valve in the context may also be valve-like structure or a tap.

Preferably, an aliquot of the amplification solution is transferred from said amplification zone to said surface hybridization zone during and/or after each amplification cycle or during and/or after every Nth amplification cycle, wherein N is 2, 3, 4, 5, 6, 7, 8, 9 or 10.

If desired, aliquots may also be transferred to the hybridization zone and/or detected in irregular intervals.

In a particularly preferred embodiment, amplification is performed in a polymerase chain reaction (PCR) and the amplification solution comprises a nucleic acid polymerase and primers substantially complementary to said target nucleic acid and wherein the amplification zone is a thermocycler zone and the temperature in said thermocycler zone is cycled between a temperature allowing for denaturation of the target nucleic acid, a temperature allowing for primer annealing to said target nucleic acid and a temperature allowing for primer extension, wherein said target nucleic acid serves as a template.

The terms "thermocycler zone" and "PCR amplification zone" are used synonymously herein.

Preferably, the transfer of an aliquot is performed during or after the annealing phase of PCR.

In a preferred embodiment of the method, the transfer of an aliquot is not performed during the first 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, preferably the first 20, more preferably the first 30 PCR cycles.

In some embodiments of the methods of the present invention, each aliquot is transferred to a different surface hybridization zone. In preferred alternative embodiments only one surface hybridization zone is present and each aliquot is transferred to this surface hybridization zone.

Using different surface hybridization zones for each aliquot allows for a "frozen" picture of the detected amplification steps.

In an alternative embodiment of the present invention, every aliquot is transferred to the same surface hybridization zone. The surface hybridization zone may be washed (e.g. to remove aspecific binding) after the hybridization and detection of each aliquot of the amplification solution and/or before every hybridization or detection. In some particular embodiments, a cumulative signal may be detected as more and more aliquots are transferred to the same surface hybridization zone. This may under certain circumstances result in a faster detection.

In a further preferred embodiment, an additional buffering composition allowing for hybridization of oligonucleotide probes to said amplified target nucleic acid sequences is added to said amplification solution during or after the transfer into the surface hybridization.

The buffering composition may comprise for example salts and/or detergents and/or herring sperm DNA or salmon sperm DNA (single stranded bulk DNA). The components of such buffering composition ("hybridization buffer") allowing for hybridization of the amplified target nucleic acid sequences to the capture probes are known to a skilled person. The buffering composition may be a dry solid or a solution. The dry solid is preferably a dried or lyophilized hybridization buffer.

In a preferred embodiment of the invention, the dry solid is immobilized on said outlet or inside the connecting channel between the amplification zone and the surface hybridization zone and the dry solid is dissolved in said aliquot during transfer of the aliquot to the surface hybridization zone.

In other embodiments of the invention, no additional buffering composition (e.g. as a dried hybridization buffer) is added.

Herein, the temperature allowing for denaturation of the target nucleic acid is preferably in the range of from about 85 to 100 °C; the temperature allowing for primer annealing to said target nucleic acid is preferably in the range of from about 40 to 65 °C and the temperature allowing for primer extension is preferably in the range of from about 60 to 75 °C.

In a particular embodiment multiple target nucleic acid sequences are detected by at least one oligonucleotide probe substantially complementary to each target nucleic acid sequence to be detected.

Multiple target nucleic acids in the context of the present invention may be any number of different target nucleic acids detectable simultaneously or in parallel, particularly but not limited to 2, 3, 4, 5, 6, 7, 8, 9, 10, 11, 12, 13, 14, 15, 16, 17, 18, 19, 20, 21,22,23,24,25,26,27,28,29,30,31,32,33,34,35,36,40,45,48,50,52,56,55,60,64, 65, 70, 72, 75, 80, 85, 90, 95, 96, ,100 or 384 target nucleic acids. Multiple target nucleic acids hybridized to capture probes may be detected simultaneously or in parallel.

The capture probes may be for example immobilized on an inner surface of said surface hybridization zone or on the surface of beads. The beads may e.g. be magnetic beads.

In some particular embodiments, for each distinct target nucleic acid sequence at least one pair of primers is used for amplification, wherein at least one primer of the pair of primers is labeled, e.g. with a fluorescent dye. In these cases fluorescence is detected on the surface of the surface hybridization zone. In other embodiments general primers are used to amplify different targets.

Preferably, the surface hybridization zone has a temperature allowing for hybridization of said capture probes to amplified target nucleic acid sequences and wherein said temperature is substantially constant.

In a particular embodiment, the temperature of the surface hybridization zone is increased after hybridization to measure melting and/or melting curves.

Furthermore, it is preferred that the capture probes are surface-immobilized oligonucleotide probes complementary to said nucleic acid sequences and detection of the amplified target nucleic acid sequences captured to the captured sites is performed with a surface-specific detection method detecting a signal in proximity to the surface.

The target nucleic acid sequences to be amplified and detected are preferably selected from the group comprising ssDNA, dsDNA, RNA.

The nucleic acid polymerase according to the present invention may be a thermostable nucleic acid polymerase e.g. from a thermophilic organism. The nucleic acid polymerase may preferably be a DNA polymerase. The polymerase may be recombinant. Also polymerases adapted for hot-start-PCR may be used.

The thermostable nucleic acid polymerase may be for example selected from the group comprising *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerase, *Pyrococcus furiosus* (Pfu) DNA polymerase, *Pyrococcus woesei* (Pwo) DNA polymerase, *Pyrococcus kodakaraensis* KOD DNA polymerase, *Thermus filiformis* (Tfi) DNA polymerase, *Sulfolobus solfataricus* Dpo4 DNA polymerase, *Thermus pacificus* (Tpac) DNA polymerase, *Thermus eggertsonii* (Teg) DNA polymerase and *Thermus flavus* (Tfl) DNA polymerase.

A very particular embodiment, relates to a method for amplification and detection of target nucleic acid sequences, wherein the target nucleic acid is a doublestranded DNA sequence (dsDNA), wherein a pair of primers is used for amplification of the dsDNA, wherein one primer is substantially complementary to a region on one strand of the dsDNA and the other primer is substantially complementary to a region on the other strand of the dsDNA, and wherein one primer is used in excess to the other primer, and wherein the melting point of the primer used in excess is at least 5°C lower than the melting point of the other primer.

In a particular embodiment of the method amplification is performed in a polymerase chain reaction (PCR) and the polymerase chain reaction (PCR) is an asymmetric PCR or is a Linear-After-The-Exponential-PCR (LATE-PCR).

Asymmetric PCR in this context is a PCR in which the concentration of the forward primer is different from the concentration of the reverse primer in the amplification solution, i.e. one primer is used in a great excess over the other primer. This results in a higher amount of single-stranded amplified DNA as compared to conventional, symmetric PCR, since the strand to which more primer is annealed to is preferentially amplified. Higher amounts of single-strand DNA may result in higher detection efficiencies.

Linear-After-The-Exponential-PCR (LATE-PCR) uses a limiting primer with a higher melting temperature than the excess primer to maintain reaction efficiency as the limiting primer concentration decreases mid-reaction.

The composition of amplification solutions allowing for amplification reactions such as PCR is known to a skilled person.

In some embodiments, the reaction container comprises a microarray at least in said surface hybridization zone and wherein said oligonucleotide probes are immobilized on (an inner surface of) said microarray.

The methods of the present invention may in some embodiments comprise the step of cooling of the amplification solution after the last amplification step (e.g. after the last PCR cycle) to a temperature below the hybridization temperature, preferably a cooling temperature in the range of from about 0 °C to 10 °C, more preferably in the range of from about 0 °C to 4 °C, most preferably around 3 °C to 4°C. In some embodiments cooling may be performed in the thermocycler zone in other embodiments cooling may be performed in a designated cooling zone that is thermally decoupling from the other zones.

The methods of the present invention are in preferred embodiments methods for the detection of nucleic acid comprising target nucleic acid sequences in said amplification solution. Thus, the amplification solution may be a solution suspected to comprise nucleic acids containing said target nucleic acid sequences.

The particular embodiments of the methods according to the present invention are also reflected in particular embodiments of the devices of the present invention, i.e. the present invention also relates to a device for conduction the methods according to the present invention. The definitions of specific terms described herein for the methods of the invention also apply for the devices of the present invention. This is particularly true for the capture probes, reaction container, target nucleic acid (sequence) and the like.

The present invention also relates to a device for amplification and detection of target nucleic acid sequences in an amplification solution in a reaction container, comprising:

> a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences and at least one other kind of zone;
> one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences;
> a detection system that detects target nucleic acid sequences which are bound to said capture probes but does essentially not detect target nucleic acid sequences which are not bound to said capture probes; and
a transportation system for transporting the amplification solution between the zones.

In a further aspect the present invention relates to a device for amplification and detection of target nucleic acid sequences in an amplification solution in a reaction container, comprising: (a) a reaction container comprising at least two kind of zones that are thermally decoupled from each other, wherein one kind of zone is a surface hybridization zone having a substantially constant generated temperature allowing for hybridization of capture probes to complementary target sequences, wherein an inner surface of the surface hybridization zone is coated with capture probes for target nucleic acid sequences; (b) a surface-specific detection system that detects targets which are bound to said surface but does essentially not detect targets which are in solution; (c) one or more temperature controllers and one or more temperature adjusters for controlling and adjusting the temperature in the zones; and
a transportation system for transporting the amplification solution between the zones.

The device of the present invention may preferably be a device for amplification and detection of target nucleic acid sequences in an amplification solution in a reaction container, comprising:

> a reaction container comprising a first compartment for amplification of target nucleic acid sequences and one or more second compartment(s) comprising a surface hybridization zone, wherein capture probes that are substantially complementary to regions on said target nucleic acid sequences are immobilized on a surface in said surface hybridization zone and wherein;
> a surface-specific detection system that detects targets which are bound to said surface but does essentially not detect targets which are in solution;
> one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences; and
> a transportation system for transporting the amplification solution between the zones.

The reaction container comprises at least one compartment for containing the amplification solution. Optionally, the reaction container comprises additionally fluidic elements such as fluid channels for transporting the amplification fluid between the said compartments or pumps for defining a flow through the said fluid channels.

In preferred embodiments of the device of the invention, the reaction container is comprised in a cartridge which may exchangeably be attached to the rest of the device. Such a cartridge may be a cartridge for the use in a thermocycler or in a device according to the present invention. The cartridge may in preferred embodiments be a disposable (one-way) cartridge. The temperature controllers and adjusters, e.g. the heaters and/or coolers are preferably not part of such a cartridge, particularly of a disposable cartridge. In alternative embodiments heaters and/or coolers may be part of the cartridge. The cartridge may comprise additional compartments, e.g. for preparation of the amplification solution (i.e. preparation of the sample). Also comprised in such a cartridge may in some embodiments be the transportation systems or parts of the transportation systems, e.g. microfluidic systems. The structure or pattern of the zones of the reaction chamber may be reflected in the structure or pattern of the heaters and/or coolers of the temperature control and adjustment system. Also the cartridge may have patterns of heat conducting and/or insulating surfaces.

It is preferred that the reaction container is comprised in an exchangeable cartridge. As stated above, the temperature controllers and/or adjusters or parts thereof is in some embodiments comprised within the cartridge or is in other embodiments not comprised within the cartridge. The same applies, independently, to the transportation system. The transportation system or parts thereof is comprised within the cartridge or is not comprised within the cartridge.

The temperature zones of the reaction container are a product of the layout of the temperature control and adjustment system, i.e. the arrangement of the heaters, coolers, heat transfer means and the like. If a cartridge is present, the layout of the zones is dependent on the design of the cartridge and the arrangement of the heaters, coolers and heat transfer means. Thus, in some embodiments the zone layout within the cartridge is defined by the layout of the temperature controllers and adjusters outside the cartridge.

Preferably, the device is a device for amplification and detection of target nucleic acid sequences in a polymerase chain reaction (PCR), wherein the amplification zone is a thermocycler zone of which the temperature can be cycled in the range of from the melting point (melting or freezing temperature) to the boiling point (boiling temperature) of the amplification solution (preferably within the range of from about 0°C to 100°C).

More preferably, the detection is a real-time detection and the PCR is a real-time PCR. More preferably, the PCR is a quantitative PCR and the amplified target nucleic acid sequences are quantified.

The temperature controllers and adjuster may for example be heaters and/or coolers, preferably with a temperature detector and/or a feedback-regulation system. The heaters may in some embodiments be e.g. Peltier elements, heating electrodes, hot/cool air devices, water baths and the like. However, the invention is not limited to these heaters/coolers and all kind of heating and cooling means may be used for controlling, adjusting and maintaining the temperature of the zones. Also combinations of different heaters and/or coolers may be used.

The device may also comprise a controller for opening or closing the tap or valve and an opening or closing mechanism for the tap or valve. By opening and closing the tap or valve and by the time of opening the valve or tap, the transfer of the amplification solution between the zones, particularly between the thermocycler zone and the surface hybridization may be controlled.

In a particular embodiment the device comprises multiple different capture probes substantially complementary to multiple target nucleic acid sequences and a detection system for simultaneously detecting multiple different targets.

According to a very preferred embodiment of the invention at least one surface hybridization zones is connected to said amplification zone through outlets comprising a valve and the device additionally comprises a controller for controlling and opening said valve.

A valve in this context may also be a valve-like structure or a tap.

The transportation system of the device may preferably be selected from the group comprising a pump, a heating element and a Microelectromechanical system (MEMS).

The transport may be an active transport, e.g. by applying a pressure with a pump or may be a passive transport, e.g. diffusion or transportation driven by capillary forces.

By controlling the transportation system, e.g. the rate of transportation and/or the periods where the transportation system is active, the amplification and detection can be further controlled. The rate of transportation, e.g. the flow from the amplification/thermocycler zone to the surface hybridization zone (e.g. in terms of volume per time) or the time the amplification solution stays in a particular zone may be controlled and adjusted according to a particular application. A skilled person is for instance aware of the timing of PCR cycles and the phases of each cycle.

Preferably, the detection system of the device is a confocal or evanescent detection system and/or is selected from the group of fluorescence detection system, CCD chip, plasmonics (surface plasmon resonance system), total internal reflection system and wire grid biosensor system.

In a particular embodiment the device comprises a microarray at least in said surface hybridization zone and said oligonucleotide probes are immobilized on an inner surface of said microarray.

The device may additionally comprise a cooling zone for cooling the amplification solution to a temperature below the hybridization temperature, preferably a temperature in the range of from about 0 °C to 10 °C, more preferably in the range of from about 0 °C to 4 °C, most preferably around 3 °C to 4°C. The amplification solution may for example be transferred to the cooling zone after the last PCR cycle.

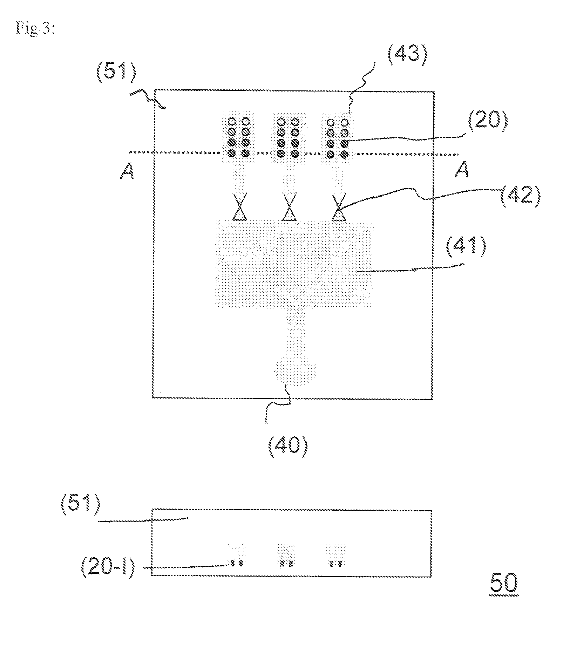

The present invention also relates to a (disposable) cartridge (50) for amplification and detection of target nucleic acid sequences in an amplification solution in a reaction container, comprising a reaction container for receiving an amplification solution comprising said target nucleic acid sequences, wherein the reaction container comprises a first compartment for amplification of target nucleic acid sequences and one or more second compartment(s) comprising a surface hybridization zone, wherein capture probes that are substantially complementary to regions on said target nucleic acid sequences are immobilized on a surface in said surface hybridization zone. An illustrative embodiment of the cartridge is shown in Fig. 3, wherein the body housing confines the reaction container which comprises one or more compartments with surface hybridization zones (43) and another compartment comprising an amplification zone (e.g. thermocycling zone, particularly for performing PCR). The body housing (51) of the cartridge preferably comprises an at least partly transparent material to enable optical detection. The cartridge is preferably made of (but is not limited to) plastic and like materials or glass. The cartridge can also be an assembly of a transparent substrate with capture probes and a box containing fluid channels. Capture probes (20) that are substantially complementary to target nucleic acid sequences are immobilized in hybridization zones. The cartridge of this embodiment preferably also comprises an inlet for fluid (40). Typically, but by no means limiting, the capture probe sites are spots of about 0.1-0.3 mm in diameter. Preferably, the reaction container comprises one compartment (41) for amplification (e.g. thermocycling in PCR) and at least one other compartment (43) comprising the surface hybridization zone with surface-immobilized capture probes (20). Typically the volume of the compartment (41) for amplification is between 1-100 μl, preferably the volume of compartment (41) is less than 50 μl. Preferably, the volume of a compartment (43) for hybridization is a small fraction of the volume of the compartment (41) for amplification, typical volumes of compartment (43) are around 1 μl, preferably less than 1 μl. A particular embodiment of such a cartridge is illustrated in appended Fig. 3.

As mentioned above, the cartridge in this preferred embodiment may be exchangeable. The device is illustrated in Fig. 4 without the cartridge and in Fig. 5 with the cartridge. The device of Fig. 4 is ready to receive a (disposable) cartridge with the reaction chamber. The device comprises a sample holder (70) for receiving the cartridge (50), heaters (80) (or coolers or other temperature adjusters) for defining the temperature in the compartments for amplification (41) and hybridization (43). A detection system ("reader") for detecting the target nucleic acids that are hybridized to the capture probes (20) is also present. A simplified schematic illustration of a scanning unit for confocal detection is shown in Figs. 4 and 5. In a particularly preferred case the scanning unit comprises an illumination/collection (of the fluorescence) lens/objective (92) and a dichroic mirror (91) that reflects the excitation light (101 in Fig. 5) resulting in excitation light directed towards the cartridge. The same lens (92) collects in this case the fluorescence (201 in Fig. 5) generated by the labeled target molecule and directs this fluorescence via the dichroic mirror (91), which transmits the fluorescent light, towards a detector (not shown in the Figures).

In a further aspect the present invention relates to a device for receiving the cartridge of the present invention, comprising:

one or more temperature controllers and/or temperature adjusters for generating a temperature profile of at least two kind of temperature zones in said reaction container, wherein one kind of zone is essentially overlapping with said surface hybridization zones, and wherein the amplification zone has (a) temperature(s) allowing for amplification of nucleic acids, and wherein the surface hybridization zone has a substantially constant generated temperature allowing for hybridization of capture probes to complementary target nucleic acid sequences;
a detection system that detects target nucleic acid sequences which are bound to said capture probes but does essentially not detect target nucleic acid sequences which are not bound to said capture probes; and
a transportation system for transporting the amplification solution between the compartments ((41) and (43)) of the cartridge; and
a receiving element for said cartridge.

The present invention also relates to the use of the methods, cartridges and devices of the present invention for quantitative analysis of target nucleic acid sequences, for simultaneous quantitative analysis of multiple target nucleic acid sequences or for analyzing a sample for the presence of a target nucleic acid.

Preferably, the methods, cartridges and devices described herein are used for clinical diagnosis, point-of care diagnosis, bio-molecular diagnostics, gene or protein expression arrays, environmental sensors, food quality sensors or forensic applications.

The present invention also relates to the use of the methods, cartridges and devices of the present invention in PCR, quantitative PCR, multiplex PCR, real-time PCR or real-time multiplex PCR, particularly quantitative real-time PCR or quantitative real-time multiplex PCR.

Amplification may be performed by various enzymatic methods including PCR (polymerase chain reaction), NASBA (nucleic acid sequence based amplification), TMA (transcription mediated amplification), and rolling circle amplification. Enzymatic amplification methods suitable for the present invention are known to a person skilled in the art.

Amplification of target nucleic acid sequences according to the present invention is performed in an amplification solution. The amplification solution may in the context of the present invention for example be a sample containing or suspected to contain nucleic acids comprising target nucleic acid sequences. A skilled person knows how to prepare a sample for performing an amplification reaction therein or knows additional components (e.g. buffers, enzymes, nucleotides, salts etc.) that have to be added to a sample in order to perform an amplification reaction in a sample. An amplification solution herein may also relate to a reaction buffer comprising nucleotides (e.g. dNTPs) and other substances (e.g. buffering agents, salts like magnesium salts, inert proteins like BSA) allowing for amplification of target nucleic acids. A skilled person knows suitable solutions (and particularly suitable concentrations of the ingredients of such solutions) for amplification reactions, particularly polymerase chain reactions (PCR). PCR comprises the repetition of cycles comprising a denaturation phase of the nucleic acid to be amplified ("target nucleic acid") at a high temperature well above the melting temperature of the target nucleic acid sequence, an annealing phase at a temperature allowing for annealing of nucleic acid primer to said target nucleic acid and a primer extension phase (elongation phase) at a temperature allowing for primer extension by a nucleic acid polymerase in which the annealed primer(s) is/are extended and the target nucleic acid serves as a template. The skilled person is aware that for polymerase chain reaction suitable primers or pairs of primers are present during amplification in the amplification solution and knows how to design such primers in order to amplify target nucleic acid sequences.

A primer is a nucleic acid strand, or a related molecule that serves as a starting point for nucleic replication. The term "nucleic acid polymerase" refers to an enzyme that synthesizes nucleic acid stands (e.g. RNA or DNA) from ribonucleoside triphosphates or deoxynucleoside triphosphates.

A thermally decoupled zone in the context of the present invention relates to a zone or a compartment of a reaction container in which the temperature can be controlled, adjusted and maintained independently from other zones or compartments or other kinds of zones or compartments of the reaction container. However, as described herein below in more detail, in some embodiments, zones of the same kind of zone may be thermally coupled or in other embodiments be thermally decoupled from each other, i.e. the temperature of zones of the same kind may be controlled, adjusted and maintained concertedly or independent from each other. That may in some embodiments mean that zones of the same kind have about the same temperature, in other embodiments it may mean that the temperature may vary among the zones of the same kind. According to the present invention, the temperatures of the different kinds of zones are controlled, adjusted and maintained independently from other kinds of zones such that the different kinds of zones are thermally-decoupled.

A kind of zone is a class of zones which serve for the same purpose. Kinds of zones are, according to the present invention, e.g. surface hybridization zones, thermocycler zones, denaturation zones, extension zones and/or annealing zones. The temperature of the hybridization zones, the extension zones, the annealing zones and the denaturation zones are substantially kept constant in the preferred embodiments of the invention during amplification and/or detection. The temperature of the thermocycler zones is changed, e.g. cycled between two, three, four or more distinct temperatures in a programmable manner during the amplification reaction. A skilled person knows how to select and/or program thermocycles for e.g. polymerase chain reaction, e.g. how to select the times and temperatures of the phases and the number of cycles. Preferably, the temperature in a thermocycler zone is cycled between denaturation temperature, annealing temperature and extension temperature. In some embodiments, e.g. for hot-start PCR, additional temperature phases and/or cycles may be included in thermo-cycling, e.g. before the first cycle or after the last cycle. The temperature in the denaturation zone (s) is the denaturation temperature, i.e. a temperature allowing for denaturation of the target nucleic acid sequence. The temperature in the annealing zone(s) is the annealing temperature, i.e. a temperature allowing for annealing of primers to the target nucleic acid sequence. The temperature in the extension zone(s) is the extension temperature, i.e. a temperature allowing for primer extension by a nucleic acid polymerase, wherein the target nucleic acid sequence serves as a template. The temperature in the surface hybridization zone(s) is the hybridization temperature, i.e. a temperature allowing for hybridization of oligonucleotide probes to the target nucleic acid sequence. These temperatures depend on the properties (e.g. length or GC content) of the target nucleic acids, primers, polymerase and oligonucleotide probes and a skilled person knows how to select, determine or calculate these temperatures. Typical denaturation temperatures are in the range of from about 90 to 99 °C, preferably of from about 94 to 98°C. Typical annealing temperatures are in the range of from about 50 to 70 °C, preferably of from about 55 to 68 °C. Typical extension temperatures are in the range of from about 70 to 80 °C, preferably of from about 72 to 75 °C. Typical hybridization temperatures are in the range of from about 40 to 70 °C, preferably of from about 45 to 68 °C.

According to the present invention, surface specific detection is obtained either by a surface specific detection system and/or by surface-specific generation of the signal to be detected.

The following definitions apply to this and the other embodiments of the invention:

Surface specific detection means that the contribution to the detected signal by amplicons that are not captured (e.g. floating in the fluid on top of the surface with capture probes) is substantially suppressed, which means preferably suppressed by at least a factor 50, more preferably by at least a factor 100, and even more preferably by at least a factor 1000, while the contribution to the detected signal by captured amplicon is (substantially) not suppressed.

For fast hybridization the following boundary conditions may apply on the part of the PCR chamber containing the capture probes: The width of the channel is proportional to the spot lateral size and the height of the channel is small (preferably about 100-200 $\mu$m).

These boundary conditions insure that all sample is passed along the capture probes and in combination with the small height of the channel provide fast hybridization of the amplicons to the capture probes.

After each PCR cycle the amount of amplicons substantially doubles and therefore the amount of hybridized amplicons increases proportionally. The amount of bound amplicons is proportional to its concentration in the solution according to well-known formula

$$ h = \frac{k_f c_A C_P}{k_f c_A + k_r} \left( 1 - e^{-(k_f c_A + k_r)t} \right) $$

Where:

$h$ - is the surface concentration of hybridized complex to the capture sites in (mol/m$^2$),
$c_A$ - analyte concentration (mol/m$^3$)
$C_P$ - capture probe surface concentration (mol/m$^2$) and $k_f$ and $k_r$ are the on and off rates of binding.

As an example the case of a fluid cell having a height of 500 microns and containing labeled primers at a concentration of 1 micromolar is considered:

1) Without surface specific detection, the labeled primers give rise to a detected signal equivalent to ~300000 labels per square micrometer. For a capture probe density of 10000 capture probes per square micrometer, in the best case this would result in a signal over background ratio of 1/30. For a typical hybridization experiment not all capture probes have bound to a labeled amplicon and the signal over background ratio will be even smaller; e.g., 1/300 for 1000 captured amplicons per square micrometer.
2) With surface specific detection, the labeled primers in solution give rise to a substantially lower detected signal; e.g., detected signal equivalent to -300 labeled primers per square micrometer for a background suppression factor of 1000. With surface-specific detection, the signal over background ratio is substantially improved to about 3 for 1000 captured labeled amplicons per square micrometer.

Another way to describe surface specific detection is to describe a surface-specific detector that detects those labels which are bound to the surface but does essentially not detect labels which are in solution. This means that the above definition given for surface specific detection applies equally to the term "a surface-specific detector that detects those labels which are bound to the surface but does essentially not detect labels which are in solution"

The capture probe molecule can be a DNA, RNA, PNA (peptide nucleic acid), LNA (locked nucleic acid), ANA (arabinonucleic acid), or HNA (hexitol nucleic acid) oligonucleotide. RNA, PNA, LNA, and HNA are able to form heteroduplexes with DNA that are more stable that DNA:DNA homoduplexes. This ensures enhanced discrimination ability for sequence mismatches (more specific hybridization). The higher stability of heteroduplexes also allows the use of shorter oligonucleotide probes at a given temperature reducing the chance of non-specific binding. PNA:DNA duplexes are formed independent of ionic strength of the hybridization buffer. This may enhance the hybridization efficiency in low salt PCR buffers.

Hybridization of a portion of the amplicons means that the concentration of amplicon can be directly calculated from the intensity of the signal measured due to hybridization of amplicons to the capture probes. If the relation between the measured signal and amplicon concentration is not linear, a correction algorithm or calibration curve may be applied in order to deduce the amplicon concentration.

The capture portion of the capture probe may contain from 10 to 200 nucleotides, preferably from 15 to 50 nucleotides, more preferably from 20 to 35 nucleotides specific for the amplicons produced during the amplification reaction. The capture probe can also contain additional nucleotide sequences that can act as a spacer between the capture portion and the surface or that can have a stabilizing function that can vary from 0 to 200 nucleotides, preferably from 0 to 50. These non-capturing nucleotide sequences can either contain normal nucleotides or a-basic nucleotides.

The capture molecule may be immobilized by its 5' end, or by its 3' end.

For multiplexing, capture molecules are immobilized in specifically localized areas of a solid support in the form of a micro-array of at least 4 capture molecules per $\mu$n$^2$, preferably at least 1000 capture molecules per $\mu$m$^2$, more preferably at least 10000 capture molecules per $\mu$m$^2$, and even more preferably 100000 capture molecules per $\mu$m$^2$.

In a specific embodiment, the capture molecules comprise a capture portion of 10 to 100 nucleotides that is complementary to a specific sequence of the amplicons such that said capture potion defines two non-complementary ends of the amplicons and a spacer portion having at least 20 nucleotides and wherein the two non-complementary ends of the amplicons comprise a spacer end and a non-spacer end, respectively, such that the spacer end is non-complementary to the spacer portion of the capture molecule, and said spacer end exceeds said non-spacer end by at least 50 bases.

The terms "nucleic acid, oligonucleotide, array, nucleotide sequences, target nucleic acid, bind substantially, hybridizing specifically to, background, quantifying" are the ones described in the international patent application WO 97/27317 incorporated herein by reference. The term polynucleotide refers to nucleotide or nucleotide like sequences being usually composed of DNA or RNA sequences.

The terms "nucleotides triphosphate, nucleotide, primer sequence" are further those described in the documents WO 00/72018 and WO 01/31055 incorporated herein by references.

References to nucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

According to a preferred embodiment the capture probes are immobilized on the hybridization surface in a patterned array. According to a preferred embodiment the capture probes are immobilized on the surface of micro-arrays.

"Micro-array" means a support on which multiple capture molecules are immobilized in order to be able to bind to the given specific target molecule. The micro-array is preferentially composed of capture molecules present at specifically localized areas on the surface or within the support or on the substrate covering the support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and can be visualized by the detector. In one particular application of this invention, micro-arrays of capture molecules are also provided on different or separate supports as long as the different supports contain specific capture molecules and may be distinguished from each other in order to be able to quantify the specific target molecules. This can be achieved by using a mixture of beads which have particular features and are able to be distinguishable from each other in order to quantify the bound molecules. One bead or a population of beads is then considered as a spot having a capture molecule specific to one target molecules.

Micro-arrays are preferentially obtained by deposition of the capture molecules on the substrate which is done by physical means such as pin or "pin and ring" touching the surface, or by release of a micro-droplet of solution by methods such as piezo-or nanodispenser.

Alternatively, in situ synthesis of capture molecules on the substrate of the embodiments of the inventions with light spatial resolution of the synthesis of oligonucleotides or polynucleotides in predefined locations such as provided by US 5,744,305 and US 6,346,413.

It may be preferred that the PCR mixture can be enriched for single stranded amplicons by means of asymmetrical PCR (or LATE PCR: linear after the exponential). In asymmetrical PCR, unequal concentrations of forward and reverse PCR primer are used. When the concentration of the labeled primer is higher that that of the unlabelled primer, the labeled strand will be amplified at a lower rate that the unlabelled strand. This not only leads to an accumulation of the labeled strand but also directly favors the hybridization of the labeled strand to the capture probe.

The term "real-time PCR" means a method which allows detection and/or quantification of the presence of the amplicons during the PCR cycles. In real-time PCR, the presence of the amplicons is detected and/or quantified in at least one of the amplification cycles. The increase of amplicons or signal related to the amount of amplicons formed during the PCR cycles are used for the detection and/or quantification of a given nucleotide sequence in the PCR solution. In a preferred embodiment, the presence of the amplicons is detected and/or quantified in every cycle.

The term "amplicon" in the invention relates to the copy of the target nucleotide sequences being the product of enzymatic nucleic acid amplification.

Instead of labeled PCR primers, internal labeling with labeled dNTPs can be used.

The label-associated detection methods are numerous. A review of the different labeling molecules is given in WO 97/27317. They are obtained using either already labeled primer, or by enzymatic incorporation of labeled nucleotides during the copy or amplification step (WO 97/27329) [intercalators are not preferred in this invention].

Possible labels are fluorochromes which are detected with high sensitivity with fluorescent detector. Fluorochromes include but are not limited to cyanine dyes (Cy3, Cy5 and Cy7) suitable for analyzing an array by using commercially available array scanners (as available from, for example, General Scanning, Genetic Microsystem). FAM (carboxy fluorescein) is also a possible alternative as a label. The person skilled in the art knows suitable labels which may be used in the context of this invention.

In a preferred embodiment of the invention, a signal increase of the fluorescence signal of the array related to the presence of the amplicons on the capture molecule is detected as compared to the fluorescence in solution.

In a particular embodiment the differences of the detection of the fluorophore present on the array is based on the difference in the anisotropy of the fluorescence being associated with a bound molecule hybridized on the capture molecule as a DNA double helix compared to the fluorescence being associated with a freely moving molecule in solution. The anisotropy depends on the mobility of the fluorophores and the lifetime of the fluorescence associated with the fluorophores to be detected. The method assay for the anisotropy on array is now available from Blueshift Biotechnologies Inc., 238 East Caribbean Drive, Sunnyvale, CA 94089 (http: //www.blueshiftbiotech.com/dynamicfl.html).

In a particular embodiment, the detection of fluorophore molecules is obtained preferably in a timer-resolved manner. Fluorescent molecules have a fluorescent lifetime associated with the emission process. Typically lifetimes for small fluorophores such as fluorescein and rhodamine are in the 2-10 nanoscecond range. Time-resolved fluorescence (TRF) assays use a long-lived (>1000ns) fluorophore in order to discriminate assay signal from short-lived interference such as autofluorescence of the matrix or fluorescent samples which almost always have lifetimes of much less than 10 ns. Lifetime is preferably modulated by the nearby presence of another fluorophore or a quencher with which a resonant energy transfer occurs. Instruments for TRF simply delay the measurement of the emission until after the short-lived fluorescence has died out and the long-lived reporter fluorescence still persists. Fluorescence lifetime can be determined in two fundamental ways. The time domain technique uses very short pulses (picosecond) of excitation and then monitors the emission in real-time over the nanosecond lifetime. Fitting the decay curve to an exponential yields the lifetime. The frequency domain technique modulates the excitation at megahertz frequencies and then watches the emission intensity fluctuate in response. The phase delay and amplitude modulation can then be used to determine the lifetime. The frequency technique for fast and economical lifetime imaging is now available from Blueshift Biotechnologies Inc. As stated above, theses definitions apply to all of the described embodiments.

In one embodiment of the invention, primers are labeled and the hybridized labeled amplicons are detected with a surface-specific detection system that detects those labels which are bound to the surface. Surface-specific means as defined above that labels which are in solution are essentially not detected.

In a preferred embodiment the signal of the label to be detected does not change in dependence of the binding state of said multiple nucleic acid sequences.

In an even more preferred embodiment the signal to be detected is a fluorescent signal. It is preferred an "always-on label" which are preferentially small organic fluorophores, but can also be a particulate label (either fluorescent or non-fluorescent), such as nano-phosphores, quantum dots.

For detection of multiple nucleic acids multiple labels may be used, in a preferred embodiment the same label is used for detection of each of said multiple target nucleic acid sequences.

The surface-specific detection system is preferentially selected from a group comprising a confocal measurement device, a plasmonic measurement device and a device for the measurement according to evanescent detection.

As mentioned above, to be able to discriminate between hybridized amplicons and primers or amplicons in solution, it is essential to make a surface specific measurement. A surface specific measurement only detects labels that are very close to the capture surface. Because hybridization can only take place where capture probes have been deposited and the PCR mixture is homogeneous, it is possible subtract the background (the fluorescence intensity between the spots) and to determine the amount of hybridized amplicons.

Possible labels are fluorescent labels or non-fluorescent (e.g. particulate) where a difference in refractive index or absorption can be detected by optical means. It should be straightforward for someone skilled in the art to know suitable fluorescent or non-fluorescent labels.

For highly surface specific measurements, that is a suppression of the background by at least a factor 50, one can distinguish between three different approaches:

1. Confocal: typical measurement height along the optical axis of 1-2 $\mu$m.
2. Plasmonic: measurement height of about the wavelength of excitation light or smaller.
3. Evanescent: measurement height of 100 nm or smaller.

1. Confocal

Confocal measurements can be made with various types of imaging equipment, e.g. a standard pinhole-based system. Such systems can be made very compact (PCT/IB2007/052499, PCT/IB2007/052634, and PCT/IB2007/052800). Different locations along the optical axis of the system give rise to different locations where the labels are imaged by the objective closest to the array surface. By using a pinhole and proper positioning -at the location where there is a sharp image of a label at the array surface- one can select a small measurement volume (depth along the optical axis of only 1-2 $\mu$m) in the neighborhood of the sensor surface.

2. Plasmonic

Here the substrate is covered with a metal such as Au, Ag. The capture probes are on the metal layer or a spacing layer is deposited on top of the metal and subsequently covered with capture probes. The fluorescence of the labels of the hybridized DNA can couple to the surface plasmon at the metal medium/fluid interface.

Labels in the fluid cannot couple or with a substantially smaller efficiency to the surface plasmon. By out coupling of the surface plasmon (that is converting the surface plasmon mode in to a propagating wave) and measuring the out coupled power, one essentially only measures the fluorescence of the labels of the hybridized DNA. As a result the fluorescence measurement is highly surface specific.

3. Evanescent

As another alternative method for enhancing the surface specificity, one can use evanescent excitation methods, where an evanescent wave is excited at the surface of the substrate and excites the fluorophores.

As a first method one can use total internal reflection (TIR) at the substrate-fluid interface, which results in measurement (excitation) volumes typically within 100-200 nm of the array surface. TIR however requires the use of a glass prism connected to the substrate or the use of a substrate with a wedge shape to enable the coupling of excitation light with angles above the critical angle of the substrate-fluid interface into the substrate.

Alternatively (as described in PCT/IB-2006/051942, PCT/IB2006/054940) one can cover the substrate with a non-transparent medium such as a metal and pattern the metal with [an array of] apertures with at least one dimension in the plane parallel to the substrate-fluid interface below the diffraction limit of light in the fluid. As an example, one can pattern the substrate with wire grids that have one in-plane dimension above and the other dimension below the diffraction limit of the light in the fluid. This results in excitation volumes within 50 nm (Measurement volumes of 20-30 nm have already been demonstrated experimentally) of the array surface. Advantage of this method over the first method [for evanescent excitation] is that it is simpler- there is no need for a prism or wedge shaped surface and that there are no special requirements for the angle of incidence and shape of the excitation spot and one can use a simple CCD camera for imaging the fluorescence- and enables substantially smaller excitation volumes.

Multiple different capture probes can be coated on the hybridization surface in a patterned array to simultaneously monitor the amplification of multiple different amplicons in the same surface hybridization zone. All capture spots monitor the amplification of a different amplicon within the same PCR mixture. This allows for much higher multiplex grades than currently possible. This allows for multiplex grades greater than 6 and up to 100 or more. An additional advantage of the embodiments of the invention is that only one fluorophore (one species) is needed which makes multiple expensive color filters and/or separated photodetectors unnecessary.

Thus, a device according to the present invention may be a micro-array.

In another embodiment of the invention the capture probes on the solid surface of the surface hybridization zone are folded probes (e.g. molecular beacons) or other probes (e.g. TaqMan probes) with a fluorescent label and quencher in close proximity to one another due to the structure of the probe. In this embodiment the PCR reaction(s) is (are) performed without any label in the reaction or label attached to the amplification primers. Specific amplicons that are formed during the PCR reaction(s) can hybridize with the labeled capture probes on the solid surface, thereby either separating quencher and label (in case of molecular beacon type folded probes) or allowing the polymerase to hydrolyze the probe (in case of TaqMan-like capture probes). As a result a fluorescent signal can be measured at the spot where the capture probes are located.

In this embodiment it is not a prerequisite to use a highly surface specific reader, since signal is only generated upon hybridization of amplicons to the capture probe. All other statements above also apply to this embodiment of the invention.

Thus, in some embodiments the capture probes are probes with a fluorescent label and a quencher in close proximity to one another due to the structure of the probes, and a signal may be detected in case an amplicon hybridizes to said capture probes.

In some embodiments, the surface hybridization zone of the device according to the present invention comprises an inner surface that is coated with capture probes for multiple target nucleic acid sequences and wherein the capture probes are labeled and a signal may be detected in case an amplicon hybridizes to said capture probes.

According to this embodiment of the device the label is preferentially selected from a group comprising molecular beacons, intercalating dyes, TaqMan probes.

In yet another embodiment of the device of the present invention the capture probes are immobilized on individually identifiable beads. In a preferred embodiment different beads have different capture probes.

It may be preferred that the beads are brought to the surface of the surface hybridization zone and captured amplicons are measured by surface-specific detection. The beads may be brought to the surface e.g. by magnetic actuation.

In a preferred embodiment the fluorescence of a label is detected. As outlined above, a person skilled in the art knows further fluorescent and non-fluorescent labels.

If the capture probes are immobilized on beads, this allows for a quasi-homogeneous assay as the beads are freely dispersed in the reaction solution. Quasi-homogeneous assays allow for faster kinetics than non-homogeneous assays. The beads can have a diameter between 50 nm and 3 $\mu$m and are individually identifiable e.g. by color-coding, bar-coding or size. Multiple beads containing different capture probes may be present in the same reaction solution. Beads with captured amplicons on them, can be brought to the surface by magnetic actuation (when (para)magnetic beads are used) or by dielectrophoresis (when non-magnetic beads are used). On the surface the beads can be identified and the captured amplicons can be detected by a surface-specific optical measurement.

Another method to distinguish different beads is based on the differences in resonance wavelength of the resonator modes propagating along the perimeter of the bead due to size differences. In this case the bead acts as a resonator that supports resonator modes propagating along the perimeter of the bead (for a spherical bead these resonator modes are so-called whispering gallery modes). The resonator is in-resonance for a wavelength where the phase shift after one roundtrip along the perimeter is a multiple of 2*pi. These resonator modes also have an evanescent field that extends into the environment (fluid) of the bead. A fluorophore in the near field region (typically <100 nm) of the bead can couple a fraction of it's fluorescence to the resonator modes supported by the bead. The fraction coupled to the resonator mode is stronger for on-resonance wavelengths than for other wavelengths and this results in a modulation of the fluorescence spectrum with the resonance peaks corresponding to the resonator modes or whispering gallery modes. The typical diameter of the beads is larger than 1 $\mu$m up to 50 $\mu$m. Detection can be performed throughout the whole volume, because the fluorescence due to fluorophores that are not bound to the bead have the intrinsic spectrum of the fluorophore, and there is no need for a surface-specific optical measurement.

Thus, the surface hybridization zone of a device according to the present invention comprises individually identifiable beads wherein capture probes for multiple target nucleic acid sequences are immobilized on said individually identifiable beads.

In a preferred embodiment the device further comprises means for bringing the beads to the surface of the surface hybridization zone and a surface-specific detector that detects those labels which are bound to the surface but does essentially not detect labels which are in solution.

In some embodiments of the invention, the reaction container is comprised in a cartridge. Such a cartridge may be a cartridge for the use in a thermocycler or in a device according o the present invention. The cartridge may in some embodiments be a disposable (one-way) cartridge.

The embodiments of the present invention have the advantage that the time for detection of hybridized amplicons is not limited by the time available during a PCR cycle.

The methods and devices of the present invention are **characterized in that** hybridization and detection are performed in a designated surface hybridization zone. This has the advantage that the conditions for hybridization and detection can be adjusted independently from the amplification process. The thermal requirements of e.g. a PCR process and surface hybridization are very different. The PCR amplification process requires (fast) temperature cycling wherein part of the cycle the sample is above the dsDNA melting temperature. On the other hand the surface hybridization needs to take place at a temperature below the nucleic acid melting point, and for optimum performances diffusion limited local depletion of amplicons above the capture probe cites should be avoided.

The present multiple-zone concept allows decoupled optimization of these two processes, the volume amplification and the surface hybridization, e.g. to provide optimal chemical (i.e. buffer) conditions and optimal temperature conditions for amplification and hybridization/detection. Additionally, the detection is very accurate due to the relatively long time available for hybridization in each cycle. Some embodiments of the present invention provide a highly flexible method, as hybridization and detection can e.g. be performed from a certain number of cycle on or every $N^{th}$ circle.

The embodiments comprising no thermocycler zone and thus no rapid temperature changes have the additional advantage that they have a reduced risk of damage to the reaction container, cartridge and/or device due to a reduced temperature induced strain.

The methods and devices of the present invention have the advantage over conventional methods and devices for nucleic acid amplification/detection (e.g. endpoint PCR, where detection and quantification is only performed after the last amplification cycle) that a quantification of the initial amount of nucleic acid is possible (e.g. in terms of copy number of DNA molecules).

In general, the methods and devices of the present invention allow for a more sensitive detection of amplified target nucleic acid sequences as compared to state of the art methods and devices, since the tapping of samples from the amplification solution during amplification allows for longer hybridization times without significantly extending the overall amplification time.

## Claims

1. Method for amplification and detection of target nucleic acid sequences in an amplification solution in a reaction container, comprising the steps of

   providing a reaction container comprising at least one surface hybridization zone in which capture probes are immobilized on a surface, wherein said capture probes are substantially complementary to regions on said target nucleic acid sequences;

   adding the amplification solution to said reaction container;

   generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences;

performing an amplification of target nucleic acid sequences in the reaction container; and

detecting amplified nucleic acid sequences in periodic or defined intervals during and/or after amplification, wherein amplified nucleic acid sequences are detected by hybridization of capture probes to said amplified nucleic acid sequences in said surface hybridization zone.

2. Method according to claim 1 for amplification and detection of target nucleic acid sequences in an amplification solution in a reaction container, comprising the steps of

providing a reaction container comprising at least one surface hybridization zone in which capture probes are immobilized on a surface, wherein said capture probes are substantially complementary to regions on said target nucleic acid sequences;

adding the amplification solution to said reaction container;

generating a temperature zone profile in the reaction container with at least two kinds of thermally decoupled zones, wherein one kind of zone is identical or at least overlapping to/with the surface hybridization zones, wherein the surface hybridization zones have a temperature allowing for hybridization of the capture probes to the target nucleic acid sequences, and the other kind of zone has (a) temperature(s) allowing for amplification of the target nucleic acid sequences;

performing an amplification of target nucleic acid sequences in the amplification zone;

transferring an aliquot of said amplification solution at periodic or defined intervals during and/or after amplification from the amplification zone to said surface hybridization zone; and

detecting amplified nucleic acid sequences in periodic or defined intervals during and/or after amplification, wherein amplified nucleic acid sequences are detected by hybridization of capture probes to said amplified nucleic acid sequences in said surface hybridization zone.

3. Method according to claim 2, wherein said amplification zone is connected to one or more surface hybridization zones through one or more outlets comprising a valve, wherein opening of the valve or the tap results in a transfer of an aliquot from the thermocycler zone to a surface hybridization zone.

4. Method according to claims 2 to 4, wherein amplification is performed in a polymerase chain reaction (PCR) and the amplification solution comprises a nucleic acid polymerase and primers substantially complementary to said target nucleic acid and wherein the amplification zone is a thermocycler zone and the temperature in said thermocycler zone is cycled between a temperature allowing for denaturation of the target nucleic acid, a temperature allowing for primer annealing to said target nucleic acid and a temperature allowing for primer extension, wherein said target nucleic acid serves as a template.

5. Method according to any one of the claims 2 to 4, wherein each aliquot is transferred to a different surface hybridization zone or alternatively wherein only one surface hybridization zone is present and each aliquot is transferred to this surface hybridization zone.

6. Method according to any one of the claims 2 to 5, wherein an additional buffering composition allowing for hybridization of oligonucleotide probes to said amplified target nucleic acid sequences is added to said amplification solution during or after the transfer into the surface hybridization.

7. Method according to any one of the claims 1 to 6, wherein multiple target nucleic acid sequences are detected by at least one oligonucleotide probe complementary to each target nucleic acid sequence to be detected.

8. Method according to any one of the claims 1 to 7, wherein the capture probes are surface-immobilized oligonucleotide probes complementary to said nucleic acid sequences,

and wherein detection of the amplified target nucleic acid sequences captured to the captured sites is performed with a surface-specific detection method detecting a signal in proximity to the surface.

9. Method according to any one of the claims 1 to 8, wherein amplification is performed in a polymerase chain reaction (PCR) and wherein the PCR is an asymmetric PCR or is a Linear-After-The-Exponential-PCR (LATE-PCR).

10. A device for amplification and detection of target nucleic acid sequences in an amplification solution in a reaction container, comprising:

a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences and at least one other kind of zone;

one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences; a detection system that detects target nucleic acid sequences which are bound to said capture probes but does essentially not detect target nucleic acid sequences which are not bound to said capture probes; and a transportation system for transporting the amplification solution between the zones.

11. Device according to claim 10 for amplification and detection of target nucleic acid sequences in an amplification solution in a reaction container, comprising:

a reaction container comprising a first compartment for amplification of target nucleic acid sequences and one or more second compartment(s) comprising a surface hybridization zone, wherein capture probes that are substantially complementary to regions on said target nucleic acid sequences are immobilized on a surface in said surface hybridization zone and wherein; a surface-specific detection system that detects targets which are bound to said surface but does essentially not detect targets which are in solution; one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences; and a transportation system for transporting the amplification solution between the zones.

12. Device according to claim 11, wherein said reaction container is comprised in an exchangeable cartridge.

13. Device according to claim 12, wherein the temperature controllers and/or adjusters or parts thereof are comprised within the cartridge.

14. Device according to any one of the claims 10 to 13, comprising multiple different capture probes substantially complementary to multiple target nucleic acid sequences and a detection system for simultaneously detecting multiple different targets.

15. Device according to any one of the claims 10 to 14, wherein said at least one surface hybridization zone is connected to said amplification zone through outlets comprising a valve and the device additionally comprises a controller for controlling and opening said valve.

16. A cartridge for amplification and detection of target nucleic acid sequences in an amplification solution in a reaction container, comprising a reaction container for receiving an amplification solution comprising said target nucleic acid sequences, wherein the reaction container comprises a first compartment for amplification of target nucleic acid sequences and one or more second compartment(s) comprising a surface hybridization zone, wherein capture probes that are substantially complementary to regions on said target nucleic acid sequences are immobilized on a surface in said surface hybridization zone.

17. A device for receiving the cartridge of claim 16, comprising:

one or more temperature controllers and/or temperature adjusters for generating a temperature profile of at least two kind of temperature zones in said reaction container, wherein one kind of zone is essentially overlapping with said surface hybridization zones, and wherein the amplification zone has (a) temperature(s) allowing for amplification of nucleic acids, and wherein the surface hybridization zone has a substantially constant generated temperature allowing for hybridization of capture probes to complementary target nucleic acid sequences; a detection system that detects target nucleic acid sequences which are bound to said capture probes but does essentially not detect target nucleic acid sequences which are not bound to said capture probes; and a transportation system for transporting the amplification solution between the zones.; and a receiving element for said cartridge.

18. Use of a method according to claims 1 to 9 or a device according to claims 10 to 15 or 17 for quantitative analysis of target nucleic acid sequences, for simultaneous quantitative analysis of multiple target nucleic acid sequences or for analyzing a sample for the presence of a target nucleic acid.

19. Use according to claim 18 for clinical diagnosis, point-of care diagnosis, bio-molecular diagnostics, gene or protein expression arrays, environmental sensors, food quality sensors or forensic applications.

20. Use of a method according to claims 1 to 9 or a device

**EP 2 138 232 A1**

according to claims 10 to 15 or 17 or the cartridge according to claim 16 in real-time PCR or real-time multiplex PCR.

Fig 1:

Fig 2:

Fig 3:

Fig 4:

(80)

(70)

(92)

(91)

(90)

100

Fig 5:

(80)

(50)

(70)

(92)

(91)

(101)

(90)

(201)

Europäisches
Patentamt

European
Patent Office

Office européen
des brevets

## EUROPEAN SEARCH REPORT

Application Number

EP 08 15 8781

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/019902 A1 (MATHIES RICHARD A [US] ET AL) 27 January 2005 (2005-01-27) * page 4, paragraph 60 - page 5, paragraph 62; claims 1,2,4,14,20 * * page 8, paragraph 83 * * page 12, paragraph 123 - page 13, paragraph 133 * * page 15, paragraph 150 - page 16, paragraph 162 * | 1-20 | INV. B01L1/00 C12Q1/68 |
| X | WO 2007/082480 A (CAPITALBIO CORP [CN]; UNIV TSINGHUA [CN]; GUO MIN [CN]; GUAN XIAOSHENG) 26 July 2007 (2007-07-26) * paragraph [0015] - paragraph [0017] * * page 20 - page 22 * * page 28, paragraph 116 - page 29, paragraph 118; figure 8 * | 1-20 | |
| X | US 2007/092901 A1 (LIGLER FRANCES S [US] ET AL) 26 April 2007 (2007-04-26) * paragraph [0093]; figures 5,6 * * paragraph [0045] - paragraph [0047] * * paragraphs [0048], [0062] * | 16 | |
| A | * claims 1,10 * | 1-15, 17-20 | TECHNICAL FIELDS SEARCHED (IPC)  B01L C12Q |
| X | EP 1 788 095 A (EPPENDORF ARRAY TECHNOLOGIES S [BE]) 23 May 2007 (2007-05-23) * the whole document * | 16 | |
| A | WO 02/20832 A (ATONOMICS APS [DK]; WARTHOE PETER [DK]) 14 March 2002 (2002-03-14) * the whole document * | 1-20 | |

The present search report has been drawn up for all claims

| Place of search | Date of completion of the search | Examiner |
|---|---|---|
| The Hague | 13 February 2009 | Santagati, Fabio |

EPO FORM 1503 03.82 (P04C01)

## ANNEX TO THE EUROPEAN SEARCH REPORT
## ON EUROPEAN PATENT APPLICATION NO.

EP 08 15 8781

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2009

| Patent document cited in search report | | Publication date | Patent family member(s) | | Publication date |
|---|---|---|---|---|---|
| US 2005019902 | A1 | 27-01-2005 | NONE | | |
| WO 2007082480 | A | 26-07-2007 | CN | 101004423 A | 25-07-2007 |
| US 2007092901 | A1 | 26-04-2007 | NONE | | |
| EP 1788095 | A | 23-05-2007 | EP 1788096 A1 | | 23-05-2007 |
| | | | EP 1788098 A1 | | 23-05-2007 |
| WO 0220832 | A | 14-03-2002 | AU 8571701 A | | 22-03-2002 |

EPO FORM P0459

For more details about this annex : see Official Journal of the European Patent Office, No. 12/82

**EP 2 138 232 A1**

**REFERENCES CITED IN THE DESCRIPTION**

**Patent documents cited in the description**

- WO 9727317 A
- WO 0072018 A
- WO 0131055 A
- US 5744305 A
- US 6346413 B
- WO 9727329 A